(19) Europäisches Patentamt
European Patent Office
Office européen des brevets

(11) Publication number: 0 045 238
B1

(12) **EUROPEAN PATENT SPECIFICATION**

(45) Date of publication of patent specification: 01.10.86

(51) Int. Cl.[4]: **A 61 K 9/20**, A 61 K 9/48

(21) Application number: 81401121.9

(22) Date of filing: 10.07.81

# (54) Acid stabilized compositions of thieno-pyridine derived compounds and process for preventing degradation of such compounds.

(30) Priority: 29.07.80 US 173310

(43) Date of publication of application: 03.02.82 Bulletin 82/05

(45) Publication of the grant of the patent: 01.10.86 Bulletin 86/40

(84) Designated Contracting States: AT BE CH DE FR GB IT LI LU NL SE

(56) References cited:
DE-A-2 157 201
FR-A-2 315 936
GB-A-1 540 743
US-A-2 990 328

(73) Proprietor: SANOFI, société anonyme
40, Avenue George V
F-75008 Paris (FR)

(72) Inventor: Aku-Ud-Din, Tanwir Chowham
1628 Crow Court
Sunnyvale, CA 94 087 (US)

(74) Representative: Bressand, Georges et al
c/o CABINET LAVOIX 2 Place d'Estienne d'Orves
F-75441 Paris Cedex 09 (FR)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 045 238 B1

**Description**

This invention relates to the stabilization of a pharmaceutical composition containing acid salts of thieno-pyridine derivatives. The stabilization is achieved using a pharmaceutically acceptable, non-volatile organic acid, particularly citric acid.

Because of the need to facilitate manufacture, application or consumption of the drug, control of the unit dose, and ease of packaging and handling, drugs are commonly manufactured and marketed in combination with other ingredients of little or no therapeutic value. Against these considerations must be reflected the need to maintain the stability of the composition over the shelf life of the formulation in order to maintain the unit dose and to avoid any untoward effects which may arise from degradation of the drug or excipients.

Initially prepared pills and capsules of compositions containing a thieno-pyridine derived drug named ticlopidine hydrochloride (see U.S. Patent N° 4,051,141) discolored during normal storage. Analysis of these materials showed degradation of ticlopidine was responsible for the discoloration. The presence of certain excipients such as gelatin, Povidone and magnesium stearate was determined to be the initiating factor in this degradation. In order to market an efficacious and acceptable drug of this structure in the proposed formulation, a means was needed for preventing this degradation which would not interfere with the action of the drug nor have a detrimental or deleterious effect on the user.

No information in the literature deals directly with the prevention of degradation in compositions of thieno-pyridine compounds insofar as is known. Anti-oxidant and chelating additives are available in the chemical arts. However, the selection, where drug formulations are concerned, is limited by the requirement that these additives be pharmaceutically acceptable at levels needed to stabilize compounds in formulations.

One class of anti-oxidant and chelating agent additives for stabilizing organic compounds and compositions is non-volatile organic acids. For example, ascorbic acid and citric acid as well as malic acid and tartaric acid have all been used as stabilizers. Citric acid in particular has been used to stabilize fats and oils (U.S. Patents 2,197,269 and 3,294,825), hydroquinone solutions (U.S. Patent 3,855,150), and drugs such as fluocinolone acetonide (Great Britain Patent 41034/62), PGE series compounds (German Patent N° 2,353,797) and L-Dopa formulations (J7 9014—167). None of these references suggest, however, that citric acid or others of that additive class would be useful in stabilizing acid addition salts of thieno-pyridine compounds in solid dosage formulations, such as capsules and tablets.

It has been discovered that addition of non-volatile, non-toxic acidic compounds having pKa's between 2—6 when aded to acid addition salts of thieno-pyridine derived compounds in dry formulations effectively prevented discoloration under normal manufacturing and storage conditions and do not interfere with other consideration of drug efficacy.

Thus one aspect of this invention is a composition containing a pharmaceutically acceptable non-volatile acidic compound having at least one acid function selected from ascorbic acid, malic acid, tartaric acid, glycolic acid, malonic acid, citric acid, maleic acid, fumaric acid, benzoic acid, cinnamic acid, or mandelic acid, and a pharmaceutically acceptable acid addition salt of a thieno-pyridine derived compound chosen from those represented by the formula

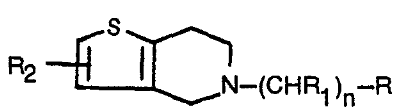

(I)

wherein:

R is phenyl or benzyl, each optionally substituted on the phenyl ring with 1 to 3 halogen atoms, alkyl of 1—6 carbon atoms, alkoxy of 1—6 carbon atoms, hydroxy or nitro;

$R_1$ is hydrogen, halogen, hydroxy or alkyl having 1—6 carbon atoms;

$R_2$ is hydrogen or halogen; and

n is 1 or 2, and when n is 2, $R_1$ may have different meanings in each $(CHR_1)$ radical,

and optionally comprising at least one pharmaceutically acceptable excipient.

Of particular interest is the compound 5-(2-chlorobenzyl)-4,5,6,7-tetrahydrothieno[3,2-*c*]-pyridine, HCl (ticlopidine-HCl).

Other pharmaceutically acceptable excipients may be present such as a lubricant, a disintegrant, an extender and a binder.

Another aspect of this invention is a process for preventing degradation of an acid addition salt of formula I type compounds, which process comprises adding a pharmaceutically acceptable non-volatile acidic compound to a dry powder formulation containing one or more said compounds and excipients, for example, a binder, a lubricant, a disintegrant and an extender.

The method of practicing this invention may be carried out by developing a formulation for acid addition salts of thieno-pyridine derived compounds represented by formula I above, which includes the drug in a pharmaceutically therapeutic amount, said pharmaceutically acceptable non-volatile acidic compound, a lubricant, a binder, a disintegrant, and a diluent. Both tablets and capsules may be prepared from this formulation.

This invention is applicable to any acid salt of a thieno-pyridine derived compound of formula I, above, with, for example, inorganic acids such as hydrochloric acid, hydrobromic acid, sulfuric acid, nitric acid, propionic acid; or organic acids such as acetic acid, propionic acid, glycolic acid, lactic acid, pyruvic acid, oxalic acid, malonic acid, succinic acid, malic acid, maleic acid, fumaric acid, tartaric acid, citric acid, benzoic acid, cinnamic acid, mandelic acid, methane sulfonic acid, ethane sulfonic acid and p-toluenesulfonic acid salicylic acid. The hydrochloric acid (HCl) salt is preferred. Generally, a therapeutically effective amount of the acid salt will be that amount necessary to give the desired pharmacological effect, and will constitute about 40—90% by weight per unit formulation. A unit formulation is a pill or capsule containing a therapeutic amount of said drug plus any added excipients.

A pharmaceutically acceptable non-volatile organic acid is one which is crystalline at room temperature and remains so throughout the range of temperatures normally encountered in the manufacture and storage of pharmaceutical compositions and has a pKa between 2—6. Of these, citric acid is preferred. Generally, the organic acid is present in an amount of 0.5—5.0% by weight.

A lubricant is generally some fatty acid derived compound or mineral oil which is blended with the formulation to lubricate the punches and die used to form pills and fill capsules. Any lubricant known to the art may be used to practice this invention, for example, magnesium stearate, calcium stearate, stearic acid, lubriwax and mineral oil but magnesium stearate is preferred. A preferred amount is 0.2—3% by dry weight.

One or more binders, in an amount of 1—5% by weight may be chosen from binders generally available such as povidone (polyvinyl pyrrolidinone), starch paste or polymers but povidone is preferred.

A disintegrant, to aid in the breaking up and disintegration of the prepared formulation, is included in this formulation in an amount of 5—15% by dry weight. Any known disintegrant may be used herein but corn starch is preferred.

Choice of a diluent or diluents is at the discretion of the practitioner but, regular lactose is preferred. It is added in the percentage needed to bring the dry powder weight to unity.

The invention is further illustrated by the following examples of the preparation of tablet and pill forms of ticlopidine HCl. These examples are by no means intended to limit the scope of this invention but are given by way of illustration.

## Example 1

### Tablets

| Ingredients | Grade | Grams per 20,000 Tab. | mg/tablet |
|---|---|---|---|
| Ticlopidine hydrochloride | | 5,000 g | 250.0 |
| Lactose, regular | USP | 1,747 | 87.35 |
| Povidone (K29—32) | USP | 156 | 7.8 |
| Citric acid anhydrous | USP | 78 | 3.9 |
| Cornstarch | USP | 780 | 39.0 |
| Magnesium stearate | USP | 39 | 1.95 |
| Total wt. | | 7,800 g | 390.0 |
| Purified water | USP | 1,350 ml | 0.0675 ml |

Tablets are prepared as follows: Ticlopidine hydrochloride and lactose are mixed in a planetary mixer for 10 minutes. Povidone and citric acid are dissolved in 1,350 ml of purified water and added slowly with continuous mixing to the drug/lactose mixture. The resultant wet granulation is mixed for 5 minutes and then passed through a number 4 or number 8 screen. The granulation is dried at 40°C to between 0.5%—1.5% moisture content and passed through a number 16 screen. The magnesium stearate and corn starch are thoroughly mixed and the mixture is blended with the dried, screened granulation and mixed for 5 minutes. If the moisture content is between 1.5%—2.5%, the granulation is compressed into tablets. As a final step the tablets are given an appropriate coating.

An example of such coating is given below for a 250 mg tablet.

| Tablet Coating Formulation | | %W/W |
|---|---|---|
| Hydroxypropyl Methylcellulose (E—5, Premium) | 2910 USP | 9.0 |
| Talc | USP | 3.0 |
| Titanium Dioxide | USP | 4.0 |
| PEG—6000 | USP | 2.0 |
| Purified Water | USP | 82.0 |
| | | 100.0 |

Comparative tests for formulations with or without citric acid (used as stabilizing agent) are given hereunder.

TABLE I

Coated tablets stored in bulk.

| Time Months | Temp. °C | Product Appearance | |
|---|---|---|---|
| | | Ticlopidine HCl tablets with citric acid (example 1) | Ticlopidine HCl tablets without citric acid (comparative example) |
| Initial | R.T.* | Round white film coated tablets | Round white film coated tablets |
| 1 | R.T. | no change | off white |
| 4.5 | R.T. | no change | Slightly yellow |
| 7.5 | R.T. | no change | Slightly yellow |

*R.T. = room temperature.

TABLE II

Coated tablets stored in an amber polystyrene bottle hermetically sealed.

| Time Months | Temp. °C | Product Appearance | |
|---|---|---|---|
| | | Ticlopidine HCl tablets with citric acid (example 1) | Ticlopidine HCl tablets without citric acid (comparative example) |
| Initial | R.T.* | as described above | as described above |
| 1 | R.T. | no change | no change |
| 3 | 40 | no change | Slightly yellow |
| 6 | 40 | no change | yellow |
| 6.5 | R.T. | no change | yellow |

*R.T. = room temperature.

Example 2

Capsules

| Ingredients | Grade | Grams per 10,000 Tab. | mg/Caps. |
|---|---|---|---|
| Ticlopidine hydrochloride | | 2,500 g | 250.0 |
| Lactose, regular | USP | 873.5 | 87.35 |
| Povidone (K29—32) | USP | 78.0 | 7.8 |
| Citric acid anhydrous | USP | 39.0 | 3.9 |
| Cornstarch | USP | 390.0 | 39.0 |
| Magnesium stearate | USP | 19.5 | 1.95 |
| Total wt. | | 3,900.0 g | 390.0 |
| Purified water | USP | 700 ml | 0.05 ml |

Capsules are prepared as follows: Ticlopidine hydrochloride and lactose are mixed in a planetary mixer for 10 minutes. Povidone and citric acid are dissolved in 700 ml of purified water and slowly added with continuous mixing to the drug/lactose mixture. Mixing is continued for 5 minutes after addition of the povidone/citric acid solution. The wet granulation is then passed through a number 4 or number 8 screen following which it is dried at 40°C to 0.5%—1.5% moisture content. This dried granulation is then passed through a number 20 screen. The magnesium stearate and corn starch are mixed and then blended with the dried granulation and mixed for 5 more minutes. The moisture content is then checked to make sure it falls between 1.5%—2.5% moisture, and then 390 milligrams per capsule are transferred into brown, N° 1 opaque gelatin capsules.

Comparative tests carried out with N° 1 size two-piece opaque tan hard gelatin capsule filled with a formulation as defined above (batch A) and with a similar formulation without citric acid (batch B). The capsules are stored in bulk.

TABLE III

| Time Months | Temp °C | Product Appearance | |
|---|---|---|---|
| | | batch A | Batch B |
| Initial | R.T. | off white powder | off white powder |
| 1 | R.T. | no change | slightly yellow |
| 4.5 | R.T. | no change | slightly yellow |
| 7.5 | R.T. | no change | yellow |

**Claims for the Contracting States: BE CH DE FR GB IT LI LU NL SE**

1. A pharmaceutical composition which comprises at least one pharmaceutically acceptable, non-volatile organic acid having at least one acid function selected from ascorbic, benzoic, cinnamic, citric, fumaric, glycolic, mandelic, malic, maleic, malonic, or tartaric acid and a pharmaceutically acceptable acid addition salt of a thieno-pyridine compound chosen from those represented by the formula

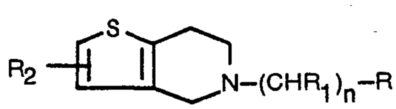

wherein:

R is phenyl or benzyl, each optionally substituted on the phenyl ring with 1 to 3 halogen atoms, alkyl of 1—6 carbon atoms, alkoxy of 1—6 carbon atoms, hydroxy or nitro;

$R_1$ is hydrogen, halogen, hydroxy or alkyl having 1—6 carbon atoms;

$R_2$ is hydrogen or halogen; and

n is 1 or 2, and when n is 2, $R_1$ may have different meanings in each ($CHR_1$) radical, and optionally comprising at least one pharmaceutically acceptable excipient.

2. The composition of claim 1, wherein said compound is the hydrochloride salt of 5-(2-chlorobenzyl)-4,5,6,7-tetrahydrothieno[3,2-*c*]-pyridine.

3. The composition of claim 2, wherein there is a plurality of excipients which include a disintegrant, a lubricant, a binder, and a diluent.

4. The composition of claim 3, wherein the lubricant is magnesium stearate, the disintegrant is corn starch, the binder is povidone (polyvinyl pyrrolidinone), and the diluent is lactose.

5. The pharmaceutical composition of claim 1 which comprises

40—90% by weight of a pharmaceutically acceptable acid addition salt of said thieno-pyridine compound;

0.5—5% by weight of said pharmaceutically acceptable, non-volatile organic acid having at least one acid function;

0.2—5% by weight of a pharmaceutically acceptable lubricant;

5—15% by weight of a pharmaceuticallly acceptable disintegrant;

1—5% by weight of a pharmaceutically acceptable binder and the remainder a pharmaceutically acceptable diluent.

6. The composition of claim 1, wherein the organic acid is citric acid.

7. The composition of claim 5, wherein said lubricant is magnesium stearate.

8. A process for preventing degradation of a pharmaceutically acceptable acid addition salt of a compound chosen from those represented by the formula

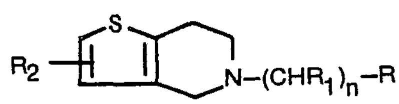

wherein:

R is phenyl or benzyl, each optionally substituted on the phenyl ring with 1 to 3 halogen atoms, alkyl of 1—6 carbon atoms, alkoxy of 1—6 carbon atoms, hydroxy or nitro;

$R_1$ is hydrogen, halogen, hydroxy or alkyl having 1—6 carbon atoms;

$R_2$ is hydrogen or halogen; and

n is 1 or 2, and when n is 2, $R_1$ may have different meanings in each ($CHR_1$) radical when formulated into a pharmaceutically acceptable composition containing at least one pharmaceutically acceptable excipient, which process comprises adding a non-volatile organic acid having at least one acid function selected from ascorbic, benzoic, cinnamic, citric, fumaric, glycolic, mandelic, malic, maleic, malonic, or tartaric acid to said composition during the formulation thereof.

9. The process of claim 8, wherein said acid is citric acid.

10. The process of claim 9, wherein said compound is the hydrochloride salt of 5-(2-chlorobenzyl)-4,5,6,7-tetrahydrothieno[3,2-*c*]pyridine.

11. The process of claim 10, wherein there is a plurality of excipients which include a disintegrant, a lubricant, a binder and a diluent.

12. The process of claim 11, wherein the lubricant is magnesium stearate, the disintegrant is corn starch, the binder is povidone (polyvinyl pyrrolidinone), and the diluent is lactose.

**Claims for the Contracting State: AT**

1. A process for preventing degradation of a pharmaceutically acceptable acid addition salt of a compound chosen from those represented by the formula

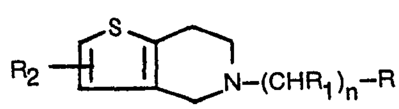

wherein:

R is phenyl or benzyl, each optionally substituted on the phenyl ring with 1 to 3 halogen atoms, alkyl of 1—6 carbon atoms, alkoxy of 1—6 carbon atoms, hydroxy or nitro;

$R_1$ is hydrogen, halogen, hydroxy or alkyl having 1—6 carbon atoms;

$R_2$ is hydrogen or halogen; and

n is 1 or 2, and when n is 2, $R_1$ may have different meanings in each ($CHR_1$) radical when formulated into a pharmaceutically acceptable composition containing at least one pharmaceutically acceptable excipient, which process comprises adding a non-volatile organic acid having at least one acid function selected from ascorbic, benzoic, cinnamic, citric, fumaric, glycolic, mandelic, malic, maleic, malonic, or tartaric acid, to said composition during the formulation thereof.

2. The process of claim 1, wherein said acid is citric acid.

3. The process of claim 2, wherein said compound is the hydrochloride salt of 5-(2-chlorobenzyl)-4,5,6,7-tetrahydrothieno[3,2-*c*]pyridine.

4. The process of claim 3, wherein there is a plurality of excipients which include a disintegrant, a lubricant, a binder and a diluent.

5. The process of claim 4, wherein the lubricant is magnesium stearate, the disintegrant is corn starch, the binder is povidone (polyvinyl pyrrolidinone), and the diluent is lactose.

**Patentansprüche für die Vertragsstaaten: BE CH DE FR GB IT LI LU NL SE**

1. Pharmazeutische Zusammensetzung, enthaltend wenigstens eine pharmazeutisch annehmbare, nicht-flüchtige organische Säure mit wenigstens einer Säure-Funktion, die aus Ascorbinsäure, Benzoesäure, Zimtsäure, Citronensäure, Fumarsäure, Glycolsäure, Mandelsäure, Äpfelsäure, Maleinsäure, Malonsäure oder Weinsäure ausgewählt ist, und ein pharmazeutisch annehmbares Säureadditionssalz einer Thienopyridin-Verbindung, die aus den durch die Formel

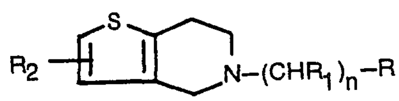

bezeichneten Verbindungen ausgewählt ist, in der

R Phenyl oder Benzyl, jeweils gegebenenfalls an dem Phenyl-Ring substituiert mit 1 bis 3 Halogen-Atomen, Alkyl mit 1 bis 6 Kohlenstoff-Atomen, Alkoxy mit 1 bis 6 Kohlenstoff-Atomen, Hydroxy oder Nitro ist,

$R_1$ Wasserstoff, Halogen, Hydroxy oder Alkyl mit 1 bis 6 Kohlenstoff-Atomen ist,

$R_2$ Wasserstoff oder Halogen ist und

n 1 oder 2 ist und, wenn n 2 ist, $R_1$ in jedem Rest ($CHR_1$) verschiedene Bedeutungen haben kann, und gegebenenfalls enthaltend wenigstens ein pharmazeutisch annehmbares Streckmittle.

2. Zusammensetzung nach Anspruch 1, dadurch gekennzeichnet, daß die Verbindung das Hydrochlorid-Salz von 5-(2-Chlorobenzyl)-4,5,6,7-tetrahydrothieno[3,2-*c*]-pyridin ist.

3. Zusammensetzung nach Anspruch 2, dadurch gekennzeichnet, daß mehrere Streckmittel vorhanden sind, zu denen ein Sprengmittel, ein Gleitmittel, ein Bindemittel und ein Verdünnungsmittel gehören.

4. Zusammensetzung nach Anspruch 3, dadurch gekennzeichnet, daß das Gleitmittel Magnesiumstearat ist, das Sprengmittel Maisstärke ist, das Bindemittel Povidone (Polyvinylpyrrolidinon) ist und das Verdünnungsmittel Lactose ist.

5. Zusammensetzung nach Anspruch 1, enthaltend

40 bis 90 Gew.-% eines pharmazeutisch annehmbaren Säureadditionssalzes der Thienopyridin-Verbindung,

0,5 bis 5 Gew.-% der pharmazeutisch annehmbaren, nicht-flüchtigen organischen Säure mit wenigstens einer Säure-Funktion,

0,2 bis 5 Gew.-% eines pharmazeutisch annehmbaren Gleitmittels;

5 bis 15 Gew.-% eines pharmazeutisch annehmbaren Sprengmittels;

1 bis 5 Gew.-% eines pharmazeutisch annehmbaren Bindemittels und

als Restmenge ein pharmazeutisch annehmbares Verdünnungsmittel.

6. Zusammensetzung nach Anspruch 1, dadurch gekennzeichnet, daß die organische Säure Citronensäure ist.

7. Zusammensetzung nach Anspruch 5, dadurch gekennzeichnet, daß das Gleitmittel Magnesiumstearat ist.

8. Verfahren zur Verhinderung des Abbaus eines pharmazeutisch annehmbaren Säureadditionssalzes einer Verbindung, die aus den durch die Formel

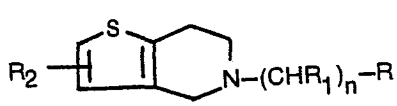

bezeichneten Verbindungen ausgewählt ist, in der

R Phenyl oder Benzyl, jeweils gegebenenfalls an dem Phenyl-Ring substituiert mit 1 bis 3 Halogen-Atomen, Alkyl mit 1 bis 6 Kohlenstoff-Atomen, Alkoxy mit 1 bis 6 Kohlenstoff-Atomen, Hydroxy oder Nitro ist,

$R_1$ Wasserstoff, Halogen, Hydroxy oder Alkyl mit 1 bis 6 Kohlenstoff-Atomen ist,

$R_2$ Wasserstoff oder Halogen ist und

n 1 oder 2 ist und, wenn n 2 ist, $R_1$ in jedem Rest ($CHR_1$) verschiedene Bedeutungen haben kann, wenn dieses in Form einer pharmazeutisch annehmbaren Zusammensetzung, die wenigstens ein pharmazeutisch annehmbares Streckmittel enthält, formuliert ist, dadurch gekennzeichnet, daß eine nicht-flüchtige organische Säure mit wenigstens einer Säure-Funktion, die aus Ascorbinsäure, Benzosäure, Zimtsäure, Citronensäure, Fumarsäure, Glycolsäure, Mandelsäure, Äpfelsäure, Maleinsäure, Malonsäure oder Weinsäure ausgewählt ist, der Zusammensetzung während der Formulierung derselben zugesetzt wird.

9. Verfahren nach Anspruch 8, dadurch gekennzeichnet, daß die organische Säure Citronensäure ist.

10. Verfahren nach Anspruch 9, dadurch gekennzeichnet, daß die Verbindung das Hydrochlorid-Salz von 5-(2-Chlorobenzyl)-4,5,6,7-tetrahydrothieno[3,2-*c*]-pyridin ist.

11. Verfahren nach Anspruch 10, dadurch gekennzeichnet, daß mehrere Streckmittel vorhanden sind, zu denen ein Sprengmittel, ein Gleitmittel, ein Bindemittel und ein Verdünnungsmittel gehören.

12. Verfahren nach Anspruch 11, dadurch gekennzeichnet, daß das Gleitmittel Magnesiumstearat ist, das Sprengmittel Maisstärke ist, das Bindemittel Povidone (Polyvinylpyrrolidinon) ist und das Verdünnungsmittel Lactose ist.

**Patentansprüche für den Vertragsstaat: AT**

1. Verfahren zur Verhinderung des Abbaus eines pharmazeutisch annehmbaren Säureadditionssalzes einer Verbindung, die aus den durch die Formel

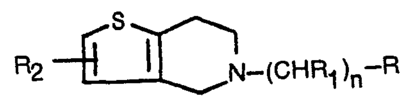

bezeichneten Verbindungen ausgewählt ist, in der

R Phenyl oder Benzyl, jeweils gegebenenfalls an dem Phenyl-Ring substituiert mit 1 bis 3 Halogen-Atomen, Alkyl mit 1 bis 6 Kohlenstoff-Atomen, Alkoxy mit 1 bis 6 Kohlenstoff-Atomen, Hydroxy oder Nitro ist,

$R_1$ Wasserstoff, Halogen, Hydroxy oder Alkyl mit 1 bis 6 Kohlenstoff-Atomen ist,

$R_2$ Wasserstoff oder Halogen ist und

n 1 oder 2 ist und, wenn n 2 ist, $R_1$ in jedem Rest $(CHR_1)$ verschiedene Bedeutungen haben kann, wenn dieses in Form einer pharmazeutisch annehmbaren Zusammensetzung, die wenigstens ein pharmazeutisch annehmbares Streckmittel enthält, formuliert ist, dadurch gekennzeichnet, daß eine nichtflüchtige organische Säure mit wenigstens einer Säure-Funktion, die aus Ascorbinsäure, Benzoesäure, Zimtsäure, Citronensäure, Fumarsäure, Glycolsäure, Mandelsäure, Äpfelsäure, Maleinsäure, Malonsäure oder Weinsäure ausgewählt ist, der Zusammensetzung während der Formulierung derselben zugesetzt wird.

2. Verfahren nach Anspruch 1, dadurch gekennzeichnet, daß die organische Säure Citronensäure ist.

3. Verfahren nach Anspruch 2, dadurch gekennzeichnet, daß die Verbindung das Hydrochlorid-Salz von 5-(2-Chlorobenzyl)-4,5,6,7-tetrahydrothieno[3,2-*c*]-pyridin ist.

4. Verfahren nach Anspruch 3, dadurch gekennzeichnet, daß mehrere Streckmittel vorhanden sind, zu denen ein Sprengmittel, ein Gleitmittel, ein Bindemittel und ein Verdünnungsmittel gehören.

5. Verfahren nach Anspruch 4, dadurch gekennzeichnet, daß das Gleitmittel Magnesiumstearat ist, das Sprengmittel Maisstärke ist, das Bindemittel Povidone (Polyvinylpyrrolidinon) ist und das Verdünnungsmittel Lactose ist.

**Revendications pour les Etats contractants: BE CH DE FR GB IT LI LU NL SE**

1. Composition pharmaceutique comprenant au moins un acide organique, non-volatil, pharmaceutiquement acceptable ayant au moins une fonction acide, choisi parmi l'acide ascorbique, l'acide benzoïque, l'acide cinnamique, l'acide citrique, l'acide fumarique, l'acide glycolique, l'acide mandélique, l'acide malique, l'acide maléique, l'acide malonique, ou l'acide tartrique et un sel d'addition avec un acide pharmaceutiquement acceptable d'un composé de thiéno-pyridine choisi parmi ceux représentés par la formule

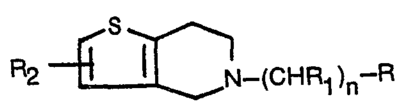

dans laquelle

R est un groupe phényle ou benzyle, chacun étant éventuellement substitué sur le groupe phényle avec de 1 à 3 atomes d'halogène, un groupe alkyle ayant de 1 à 6 atomes de carbone, alcoxy ayant de 1 à 6 atomes de carbone, hydroxy ou nitro;

$R_1$ est l'hydrogène, un halogène, un groupe hydroxy ou alkyle ayant de 1 à 6 atomes de carbone;

$R_2$ est l'hydrogène ou un halogène; et

n est 1 ou 2, et lorsque n est 2, $R_1$ peut avoir des significations différentes dans chaque radical $(CHR_1)$; et éventuellement au moins un excipient pharmaceutiquement acceptable.

2. Composition selon la revendication 1, dans laquelle le composé est le chlorhydrate de 5-(2-chlorobenzyl)-4,5,6,7-tétrahydrothiéno[3,2-*c*]-pyridine.

3. Composition selon la revendication 2, dans laquelle il y a plusieurs excipients qui comprennent un agent de désintégration, un lubrifiant, un liant et un diluant.

4. Composition selon la revendication 3, dans laquelle le lubrifiant est le stéarate de magnésium, l'agent de désintégration est l'amidon de maïs, le liant est la povidone (polyvinyl pyrrolidone) et le diluant est le lactose.

5. Composition pharmaceutique selon la revendication 1, qui comprend

de 40 à 90% en poids d'un sel d'addition avec un acide pharmaceutiquement acceptable du dit dérivé de thiéno-pyridine;

de 0,5 à 5% en poids de l'acide organique, non-volatil, pharmaceutiquement acceptable, ayant au moins une fonction acide;

de 0,2 à 5% en poids d'un lubrifiant pharmaceutiquement acceptable;

de 5 à 15% en poids d'un agent de désintégration pharmaceutiquement acceptable;

de 1 à 5% en poids d'un liant pharmaceutiquement acceptable, le reste étant un diluant pharmaceutiquement acceptable.

6. Composition selon la revendication 1, dans laquelle l'acide organique est l'acide citrique.

7. Composition selon la revendication 5, dans laquelle le lubrifiant est le stéarate de magnésium.

8. Procédé pour empêcher la dégradation d'un sel d'addition avec un acide pharmaceutiquement acceptable, d'un composé choisi parmi ceux représentés par la formule

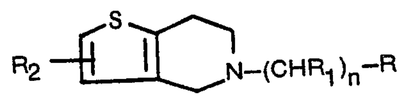

dans laquelle

R est un groupe phényle ou benzyle, chacun étant éventuellement substitué sur le noyau phényle avec de 1 à 3 atomes d'halogène, un groupe alkyle ayant de 1 à 6 atomes de carbone, alcoxy ayant de 1 à 6 atomes de carbone, hydroxy ou nitro;

$R_1$ est l'hydrogène, un halogène, un groupe hydroxy ou alkyle ayant de 1 à 6 atomes de carbone;

$R_2$ est l'hydrogène ou un halogène; et

n est 1 ou 2, et lorsque n est 2, $R_1$ peut avoir des significations différentes dans chaque radical $(CHR_1)$, lorsque le composé est formulé en une composition pharmaceutiquement acceptable contenant au moins un excipient pharmaceutiquement acceptable, lequel procédé consiste à ajouter un acide organique, non-volatil, ayant au moins une fonction acide, choisi parmi l'acide ascorbique, l'acide benzoïque, l'acide cinnamique, l'acide citrique, l'acide fumarique, l'acide glycolique, l'acide mandélique, l'acide malique, l'acide maléique, l'acide malonique ou l'acide tartrique, à la dite composition pendant sa formulation.

9. Procédé selon la revendication 8, dans lequel l'acide est l'acide citrique.

10. Procédé selon la revendication 9, dans lequel le composé est le chlorhydrate de 5-(2-chlorobenzyl)-4,5,6,7-tétrahydrothiéno[3,2-*c*]-pyridine.

11. Procédé selon la revendication 10, dans lequel il y a plusieurs excipients qui comprennent un agent de désintégration, un lubrifiant, un liant et un diluant.

12. Procédé selon la revendication 11, dans lequel le lubrifiant est le stéarate de magnésium, l'agent de désintégration est l'amidon de maïs, le liant est la povidone (polyvinyl pyrrolidone) et le diluant est le lactose.

**Revendications pour l'Etat contractant: AT**

1. Procédé pour empêcher la dégradation d'un sel d'addition avec un acide pharmaceutiquement acceptable d'un composé choisi parmi ceux représentés par la formule

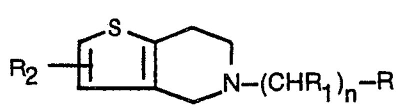

dans laquelle

R est un groupe phényle ou benzyle, chacun pouvant être éventuellement substitué sur le noyau phényle par de 1 à 3 atomes d'halogène, un groupe alkyle ayant de 1 à 6 atomes de carbone, alcoxy ayant de 1 à 6 atomes de carbone, hydroxy ou nitro;

$R_1$ est l'hydrogène, un halogène, un groupe hydroxy ou alkyle ayant de 1 à 6 atomes de carbone;

$R_2$ est l'hydrogène ou un halogène; et

n est 1 ou 2, et lorsque n est 2, $R_1$ peut avoir différentes significations dans chaque radical $(CHR_1)$, lorsqu'il est formulé dans une composition pharmaceutiquement acceptable contenant au moins un

excipient pharmaceutiquement acceptable, lequel procédé consiste à ajouter un acide organique, non-volatil, ayant au moins une fonction acide, choisi parmi les acides ascorbique, benzoïque, cinnamique, citrique, fumarique, glycolique, mandélique, malique, maléique, malonique ou tartrique, à la composition pendant sa formulation.

2. Procédé selon la revendication 1, dans lequel l'acide est l'acide citrique.

3. Procédé selon la revendication 2, dans lequel le composé est le chlorhydrate de 5-(2-chlorobenzyl)-4,5,6,7-tétrahydrothiéno[3,2-*c*]-pyridine.

4. Procédé selon la revendication 3, dans lequel il y a plusieurs excipients qui comprennent un agent de désintégration, un lubrifiant, un liant et un diluant.

5. Procédé selon la revendication 4, dans lequel le lubrifiant est le stéarate de magnésium, l'agent de désintégration est l'amidon de maïs, le liant est la povidone (polyvinyl pyrrolidone) et le diluant est le lactose.